# EUROPEAN PATENT APPLICATION

(11) **EP 2 750 059 A2**
(43) Date of publication of application: **02.07.2014**
(21) Application number: 13199349.5
(22) Date of filing: 23.12.2013
(51) Int. Cl.: G06F 19/00

(54) **Monitoring apparatus**

(30) Priority: 26.12.2012 JP 2012283021
(71) Applicant: Nihon Kohden Corporation, Shinjuku-ku Tokyo (JP); Inoue, Hiromitsu, Tokyo (JP)
(72) Inventor: Inoue, Hiromitsu, Setagaya-ku, Tokyo (JP); Kobayashi, Naofumi, Shinjuku-ku, Tokyo (JP); Suzuki, Katsuyoshi, Shinjuku-ku, Tokyo (JP); Okuno, Shinya, Shinjuku-ku, Tokyo (JP)
(74) Representative: Grünecker, Kinkeldey, Stockmair & Schwanhäusser

(57) **Abstract**

A monitoring apparatus includes: a storing section which is configured to store identification information for identifying the own apparatus from another monitoring apparatus; and a notifying section which is configured to output the identification information together with notification information indicating that an object to be monitored satisfies a predetermined condition.

## Description

### CROSS-REFERENCE TO RELATED APPLICATION(S)

This application is based upon and claims the benefit of priority from prior Japanese patent application No. 2012-283021, filed on December 26, 2012, the entire contents of which are incorporated herein by reference.

### BACKGROUND

The presently disclosed subject matter relates to a monitoring apparatus including a notifying section which performs a notification when an object to be monitored satisfies a predetermined condition.

As an apparatus of this kind, there is a monitoring apparatus which is installed in a medical institution or the like, which acquires biological information of a patient who is an object to be monitored, and which, when the biological information satisfies a predetermined condition, outputs an alarm (for example, see JP-A-2011-72568).

In a medical institution or the like, in order to mainly prevent the burden on the operator from being increased and to prevent the operator from being confused, or to facilitate the management of the institution, the institution is often configured in such a manner that monitoring apparatuses having the same function are unified in the model. Monitoring apparatuses of the same model output an alarm in the identical method. In the case where a plurality of monitoring apparatuses simultaneously output an alarm, or the case where a plurality of monitoring apparatuses of the same model are installed in the same place, therefore, it is often impossible to immediately identify the source of the alarm. This may be a cause of impeding a medical person from promptly responding to the alarm condition.

### SUMMARY

The presently disclosed subject matter may provide a technique in which, even in a situation where a plurality of monitoring apparatuses of the same model are installed, the source of an alarm can be identified easily and rapidly.

The monitoring apparatus may comprise: a storing section which is configured to store identification information for identifying the own apparatus from another monitoring apparatus; and a notifying section which is configured to output the identification information together with notification information indicating that an object to be monitored satisfies a predetermined condition.

The identification information may be output as at least sound information.

The identification information may include at least one of an apparatus ID identifying the own apparatus and an installation position of the own apparatus.

The monitoring apparatus may further comprise an information acquiring section which is configured to acquire biological information of a patient which is the object to be monitored. The installation position may be indicated by at least one of a ward number, a bed number, a position of a bed in a ward, a patient ID, and a name of a patient.

The monitoring apparatus may further comprise a displaying section which, also when the notification information is not output, is configured to display the identification information.

The identification information which is displayed on the displaying section may identify the own apparatus from the another monitoring apparatus, by means of at least one of a character, a symbol, and a color.

The monitoring apparatus may further comprise an inputting section through which the identification information is input. The storing section may updatably store the identification information.

### BRIEF DESCRIPTION OF THE DRAWINGS

Fig. 1 is a functional block diagram showing the configuration of a monitoring apparatus of an embodiment of the presently disclosed subject matter.
Figs. 2A and 2B are diagrams showing a use example of monitoring apparatuses.
Figs. 3A and 3B are diagrams showing another use example of monitoring apparatuses.
Fig. 4 is a diagram showing a further use example of monitoring apparatuses.

### DETAILED DESCRIPTION OF EXEMPLARY EMBODIMENTS

An embodiment of the presently disclosed subject matter will be described in detail with reference to the accompanying drawings. In the drawings which will be used in the following description, the scale is adequately changed in order to draw components in a recognizable size.

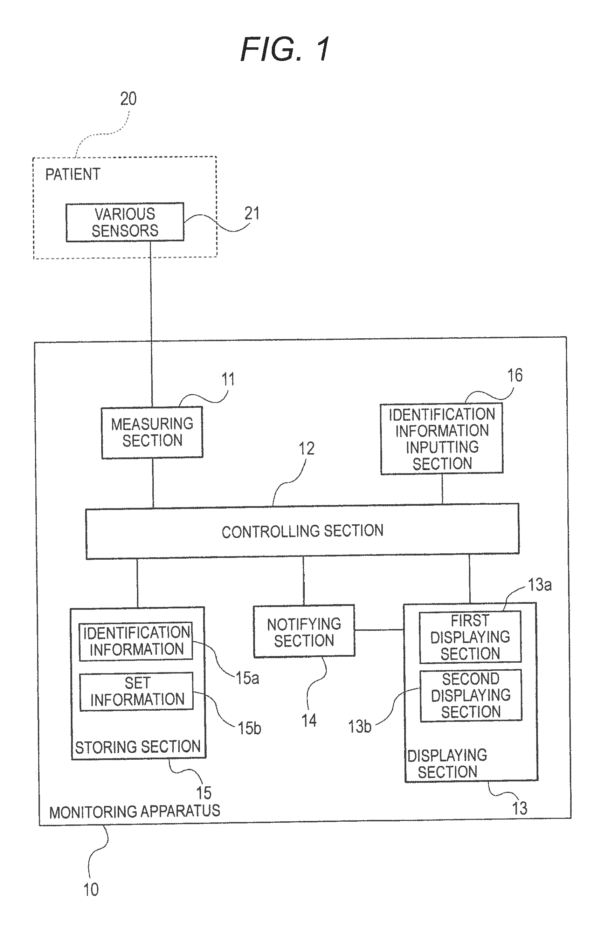

Fig. 1 is a functional block diagram showing the configuration of a monitoring apparatus 10 of an embodiment of the presently disclosed subject matter. The monitoring apparatus 10 includes a measuring section 11, a controlling section 12, a displaying section 13, a notifying section 14, a storing section 15, and an identification information inputting section 16.

Various sensors 21 such as electrodes and a cuff are attached to a patient 20 who is an object to be monitored, and measurement signals relating to various parameters indicating the condition of the patient 20 are supplied to the measuring section 11. Examples of the parameters are the heart rate, the pulse rate, the arterial oxygen saturation, the concentration of carbon dioxide in the expired gas, the blood pressure, the respiratory rate, the brain pressure, and the body temperature. The measuring section 11 which functions as the information acquiring section in the presently disclosed subject matter acquires measurement values of the parameters as biological information.

The controlling section 12 is a computation processing circuit including an arithmetic device such as a CPU, and memories such as a RAM and a ROM, and controls the overall operation of the monitoring apparatus 10. Various operations and functions of the controlling section 12 which will be hereinafter described can be realized by the operation of hardware such as circuit devices, that of software such as programs stored in the arithmetic device, or a combination of these operations.

The displaying section 13 is communicatably connected to the controlling section 12, and includes a first displaying section 13a. The first displaying section 13a is a displaying device which is disposed on the front surface of the apparatus. The controlling section 12 causes measurement signals supplied to the measuring section 11 to be displayed on the first displaying section 13a in real time.

The notifying section 14 is communicatably connected to the controlling section 12. The controlling section 12 monitors the measurement values of the various parameters which are acquired by the measuring section 11. If the measurement values satisfy predetermined conditions, the controlling section 12 determines that the patient 20 is in a condition in which medical procedures must be performed, and controls the notifying section 14 so as to output an alarm which is identification information.

Manners of outputting alarms are defined respectively for predetermined conditions in a standard. An alarm is performed by using at least one of visual notification (a display of a message, a display of a mark, light emission, or the like) and auditory notification (a beep sound, an announcement, or the like). The notifying section 14 is communicatably connected to the displaying section 13. At least a part of visual alarms is output by using the first displaying section 13a.

The storing section 15 is communicatably connected to the controlling section 12. The storing section 15 is disposed in the form of a part of the computation processing circuit constituting the controlling section 12, or an independent storage device or storage medium. The storing section 15 stores identification information 15a.

The identification information 15a is information for identifying the monitoring apparatus 10 which is the own apparatus, from other monitoring apparatuses. The identification information 15a contains an apparatus ID identifying the monitoring apparatus 10, and the installation position of the monitoring apparatus 10. The installation position is identified by at least one of the ward and bed numbers of the patient 20, the position of the bed in the ward, the patient ID, and the name of the patient.

The identification information 15a indicating the apparatus ID of the monitoring apparatus 10 is previously stored in the storing section 15. The identification information 15a indicating the installation position of the monitoring apparatus 10 is input by the user as required through the identification information inputting section 16, and stored in the storing section 15.

The identification information inputting section 16 is communicatably connected to the controlling section 12. The identification information inputting section 16 is configured as a man-machine interface in which the user inputs instructions, by buttons, switches, a mouse, a keyboard, or the like. Alternatively, at least a part of the first displaying section 13a may be configured as a touch panel which functions as a part of the identification information inputting section 16.

The controlling section 12 is configured so as to, if determining that the patient 20 is in a predetermined condition, control the notifying section 14 so as to output the identification information 15a as sound information together with an alarm. Namely, the user is notified of information for identifying the monitoring apparatus 10 from the other monitoring apparatuses, by the notifying section 14 together with the alarm condition.

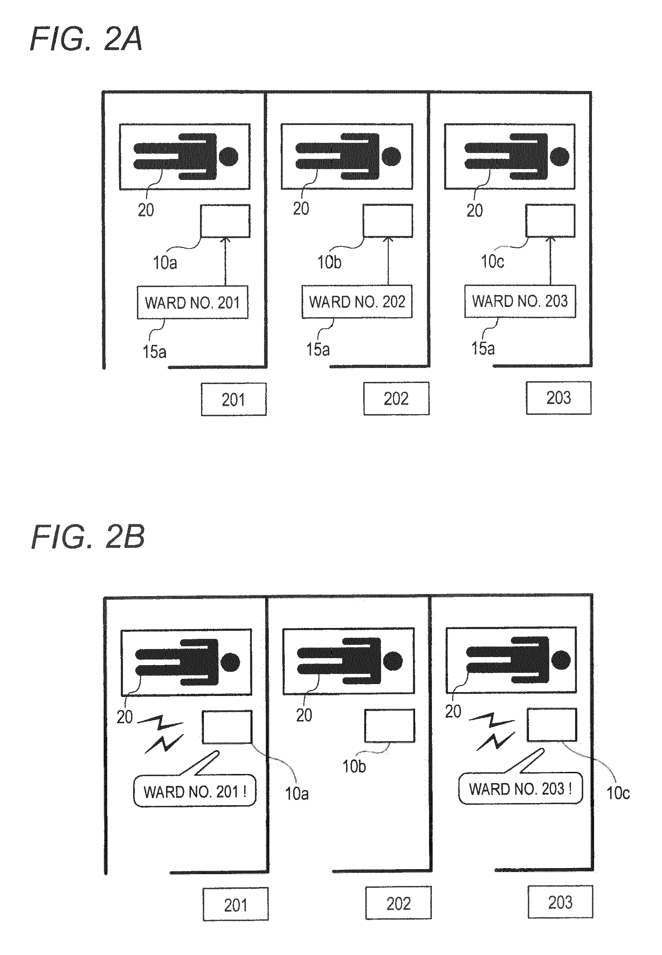

Referring to Figs. 2A and 2B, a use example of monitoring apparatuses 10 will be described. In the example, the monitoring apparatuses 10 of the same model are installed respectively in a plurality of wards (Ward No. 201, Ward No. 202, and Ward No. 203). For the sake of convenience, the monitoring apparatus installed in Ward No. 201 is denoted by 10a, that installed in Ward No. 202 is denoted by 10b, and that installed in Ward No. 203 is denoted by 10c. When each of the monitoring apparatuses 10a, 10b, 10c is installed, the user (medical person) inputs the identification information 15a through the identification information inputting section 16.

In the example, it is assumed that each of the monitoring apparatuses is identified by the ward number. As shown in Fig. 2A, as the identification information 15a, "Ward No. 201" is input in the monitoring apparatus 10a, "Ward No. 202" is input in the monitoring apparatus 10b, and "Ward No. 203" is input in the monitoring apparatus 10c. As a result, each of the monitoring apparatuses 10a, 10b, 10c is identified from the other monitoring apparatuses by means of identification of the installation position.

Fig. 2B shows a state where the monitoring apparatus 10a and the monitoring apparatus 10c output an alarm. In each of the monitoring apparatuses 10a, 10c, the notifying section 14 outputs the identification information 15a stored in the storing section 15, as sound information together with a predetermined alarm indicating the condition of the corresponding patient 20. Namely, a voice announcement "Ward No. 201" is output from the monitoring apparatus 10a, and a voice announcement "Ward No. 203" is output from the monitoring apparatus 10c.

According to the configuration, the user who receives the notification can intuitively know the monitoring apparatus of the source of the notification, and promptly rush to the patient relevant to the notification. Also in a situation where a plurality of monitoring apparatuses 10 of the same model are installed respectively in places which are relatively remote from each other, and simultaneously output an alarm, the user can easily know the respective sources of alarm notification. This effect is more pronounced in the case where the contents of the alarms which are simultaneously output are identical to each other.

The identification information 15a stored in the storing section 15 is updatable. When the user re-inputs adequate identification information 15a through the identification information inputting section 16 in accordance with the installation place of the monitoring apparatus 10, the identification information 15a can be updated. Although the same monitoring apparatus 10 is used, therefore, notification contents can be changed according to the installation place.

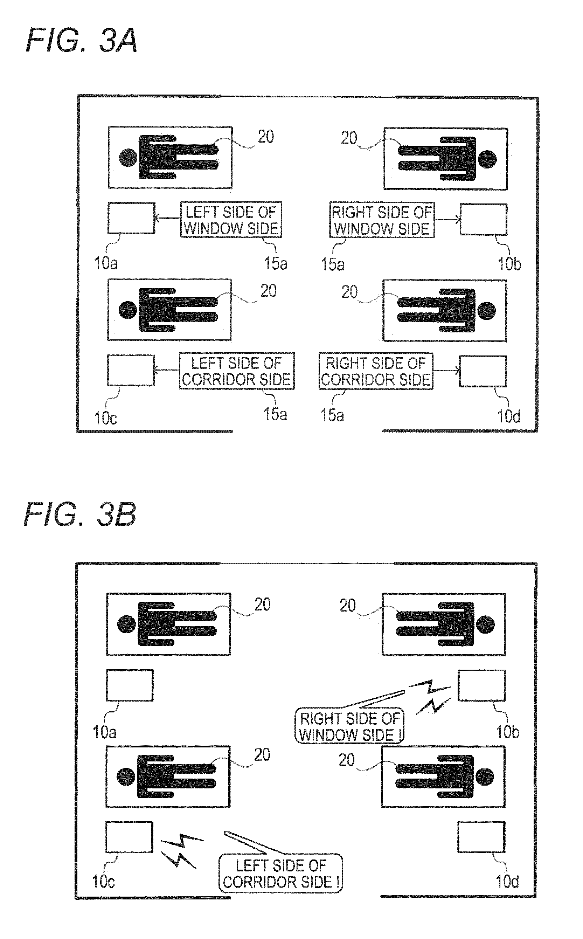

Referring to Figs. 3A and 3B, another use example of monitoring apparatuses 10 will be described. In the example, a plurality of monitoring apparatuses 10 of the same model are installed in the same ward. For the sake of convenience, the monitoring apparatus installed at the side of the left bed which is remote from the doorway is denoted by 10a, that installed at the side of the right bed which is remote from the doorway is denoted by 10b, that installed at the side of the left bed which is close to the doorway is denoted by 10c, and that installed at the side of the right bed which is close to the doorway is denoted by 10d. When each of the monitoring apparatuses 10a, 10b, 10c, 10d is installed, the user (medical person) inputs the identification information 15a through the identification information inputting section 16.

In the example, it is assumed that each of the monitoring apparatuses is identified by the position the corresponding bed in the ward. As shown in Fig. 3A, as the identification information 15a, "Left side of window side" is input in the monitoring apparatus 10a, "Right side of window side" is input in the monitoring apparatus 10b, "Left side of corridor side" is input in the monitoring apparatus 10c, and "Right side of corridor side" is input in the monitoring apparatus 10d. As a result, each of the monitoring apparatuses 10a, 10b, 10c, 10d is identified from the other monitoring apparatuses by means of identification of the installation position.

Fig. 3B shows a state where the monitoring apparatus 10b and the monitoring apparatus 10c output an alarm. In each of the monitoring apparatuses 10b, 10c, the notifying section 14 outputs the identification information 15a stored in the storing section 15, as sound information together with a predetermined alarm indicating the condition of the corresponding patient 20. Namely, a voice announcement "Right side of window side" is output from the monitoring apparatus 10b, and a voice announcement "Left side of corridor side" is output from the monitoring apparatus 10c.

Even in a situation where a plurality of monitoring apparatuses 10 are installed respectively in places which are relatively close to each other, and simultaneously output an alarm, and the contents of the alarms are identical to each other, the user can easily know the respective sources of alarm notification. This enables a prompt response to the alarm condition.

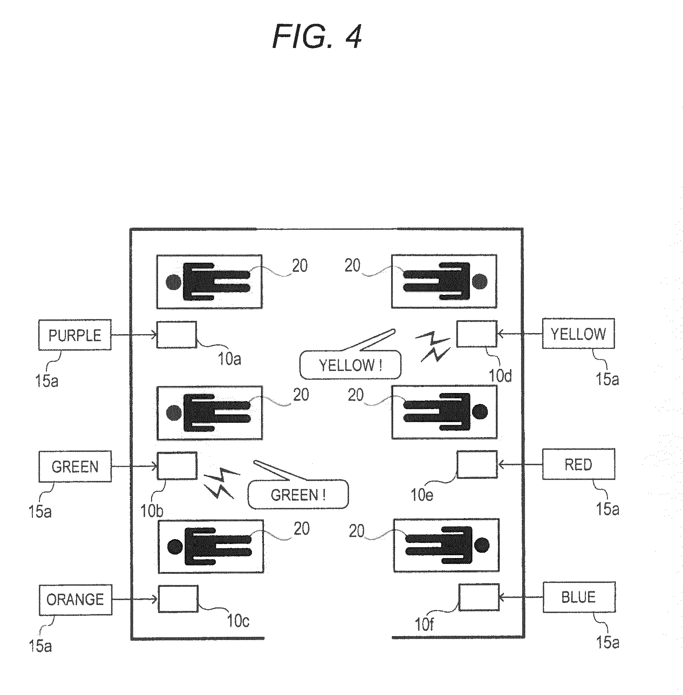

In the case where it is difficult to specifically identify the positions of beds in a ward, the installation positions of beds, i.e., those of monitoring apparatuses may be identified respectively by colors as shown in the example of Fig. 4, so that each of the monitoring apparatuses can be identified from the other monitoring apparatuses.

For the sake of convenience, the monitoring apparatuses which are installed at the sides of the beds that are located on the left side of the doorway are denoted by 10a, 10b, and 10c in descending order of the distance from the doorway, respectively. Similarly, the monitoring apparatuses which are installed at the sides of the beds that are located on the right side of the doorway are denoted by 10d, 10e, and 10f in descending order of the distance from the doorway, respectively. As identification information 15a, "PURPLE" is allocated to the monitoring apparatus 10a, "GREEN" to the monitoring apparatus 10b, "ORANGE" to the monitoring apparatus 10c, "YELLOW" to the monitoring apparatus 10d, "RED" to the monitoring apparatus 10e, and "BLUE" to the monitoring apparatus 10F.

Fig. 4 shows a state where the monitoring apparatus 10b and the monitoring apparatus 10d output an alarm. In each of the monitoring apparatuses 10b, 10d, the notifying section 14 outputs the identification information 15a stored in the storing section 15, as sound information together with a predetermined alarm indicating the condition of the corresponding patient 20. Namely, a voice announcement "Green" is output from the monitoring apparatus 10b, and a voice announcement "Yellow" is output from the monitoring apparatus 10d.

As shown in Fig. 1, the displaying section 13 of the monitoring apparatus 10 further includes a second displaying section 13b. The second displaying section 13b is disposed for displaying the identification information 15a also when an alarm is not output.

In the case shown in Fig. 4, for example, characters "Green" which are the identification information 15a are always displayed on the second displaying section 13b disposed in the monitoring apparatus 10b. During a daily medical practice, a medical person who is the user sees the characters "Green" displayed on the second displaying section 13b.

Therefore, the medical person is daily conscious of the correspondence relationship between the monitoring apparatus 10b and a voice "Green" which is output from the notifying section 14 when an alarm is generated, and identification from the other monitoring apparatuses. When an alarm is actually generated, the medical person can intuitively know the monitoring apparatus of the source of the notification, and it is possible to avoid the situation where a patient is misidentified.

The embodiment has been described in order to facilitate understanding of the presently disclosed subject matter, and is not intended to limit the presently disclosed subject matter. It is a matter of course that the presently disclosed subject matter may be changed or improved without departing the spirit thereof, and includes equivalents thereof.

The manners of setting the identification information 15a which have been described with reference to Figs. 2A to 4 may be combined with each other. In the case where a plurality of beds are placed in a plurality of wards, for example, the identification information 15a can be identified in the form of, for example, "Right side of window side of Ward No. 201". The identification information 15a may be configured by patient information (the name of the patient, the patient ID, or the like) in addition to or in place of information relating to the position.

The identification information 15a which is displayed on the second displaying section 13b may be configured by at least one of a character(s), a symbol, and a color as far as identification from the other monitoring apparatuses is enabled. In the monitoring apparatus 10b of Fig. 4, in place of the display of the characters "Green" on the second displaying section 13b, for example, a green mark may be displayed, or green light may be emitted from the second displaying section 13b. For example, another configuration may be employed where the voice announcement of the identification information 15a in the case of the generation of an alarm is output by a voice of a male or female to identify the source of the notification, and a mark indicating a male or female is displayed on the second displaying section 13b. Alternatively, the source of the notification may be identified by changing the tone or kind of the alarm sound.

A configuration may be employed where the own voice of a medical person is previously stored in the storing section 15, and the voice is used as the identification information 15a. In this case, when the announcement voice is output by using the own voice, it is possible to easily know that an abnormality occurs in a patient of whom the medical person is in charge.

The identification information 15a which is output from the notifying section 14 when an alarm is generated is not always required to be configured by sound information. In the example shown in Fig. 4, in addition to the configuration where the monitoring apparatuses 10b, 10d output voice announcements "Green" and "Yellow", respectively, for example, another configuration may be employed where, parts of the monitoring apparatuses 10b, 10d emit green light and yellow light, respectively. According to the configuration, the user can know the source of the notification more easily and surely. In a situation where a monitoring apparatus can be identified without using sound information, a configuration may be employed where identification information is output by using only visual notification.

As described above, the combination of the identification information 15a stored in the storing section 15 and the manner of notifying it can be arbitrarily set in accordance with the use conditions of the monitoring apparatus 10. The combination is set by the user through the identification information inputting section 16. As shown in Fig. 1, the set contents are stored as set information 15b in the storing section 15, and can be updated as needed.

The identification information 15a is not required to be always displayed on the second displaying section 13b. A configuration may be employed where a situation where a medical person approaches the monitoring apparatus 10 is sensed and the information is then displayed, or where the information is displayed when a medical person performs a predetermined operation as needed.

When outputting the identification information 15a as sound information, the monitoring apparatus 10 may additionally output information relating to the cause of the alarm. In the case where, in Fig. 2B, the monitoring apparatus 10a is to output an alarm indicating "bradycardia" of the patient, for example, a voice announcement "Ward No. 201, Bradycardia" may be output.

The second displaying section 13b may be configured as a part of the first displaying section 13a, or disposed in the monitoring apparatus 10 as a displaying device which is separated from the first displaying section 13a.

The operation of inputting the identification information 15a through the identification information inputting section 16 is not essential. As far as identification from the other monitoring apparatuses is enabled, a configuration may be employed where only sound information which is previously stored in the storing section 15, and which indicates the apparatus ID is output together with an alarm. The term "apparatus ID" means that the ID contains at least one of the kind, name, and model number of the monitoring apparatus 10.

The presently disclosed subject matter can be applied to an adequate monitoring apparatus including a notifying section which performs a notification when an object to be monitored satisfies predetermined conditions, in addition to the monitoring apparatus 10 for monitoring the condition of the patient 20.

According to an aspect of the presently disclosed subject matter, the information for identifying a certain monitoring apparatus from another monitoring apparatus is notified together with the notification information, and therefore the user can easily know the source of alarm notification.

According to an aspect of the presently disclosed subject matter, since the identification information includes at least one of the apparatus ID identifying the own apparatus and the installation position of the own apparatus, the user who receives the notification can know the source of the notification more intuitively.

According to an aspect of the presently disclosed subject matter, since the monitoring apparatus further includes an information acquiring section which acquires the biological information of the patient which is the object to be monitored, and the installation position is indicated by at least one of the ward number, the bed number, the position of the bed in the ward, the patient ID, and the name of the patient, a medical person can easily identify the monitoring apparatus which is the source of the notification, and promptly rush to the patient.

According to an aspect of the presently disclosed subject matter, since the monitoring apparatus further includes a displaying section which, also when the notification information is not output, displays the identification information, also in a state where the notification information is not output, a correspondence relationship between the monitoring apparatus and the identification information can be always shown to the user so that the user is conscious of the correspondence relationship.

According to an aspect of the presently disclosed subject matter, since the monitoring apparatus further includes an inputting section through which the identification information is input, and the storing section updatably stores the identification information, while the same monitoring apparatus is used, notification contents can be adequately changed in accordance with the use conditions.

## Claims

1. A monitoring apparatus comprising:
a storing section which is configured to store identification information for identifying the own apparatus from another monitoring apparatus; and
a notifying section which is configured to output the identification information together with notification information indicating that an object to be monitored satisfies a predetermined condition.

2. The monitoring apparatus according to claim 1, wherein
the identification information is output as at least sound information.

3. The monitoring apparatus according to claim 1 or claim 2, wherein
the identification information includes at least one of an apparatus ID identifying the own apparatus and an installation position of the own apparatus.

4. The monitoring apparatus according to any one of claims 1 to 3, further comprising an information acquiring section which is configured to acquire biological information of a patient which is the object to be monitored, wherein
the installation position is indicated by at least one of a ward number, a bed number, a position of a bed in a ward, a patient ID, and a name of a patient.

5. The monitoring apparatus according to any one of claims 1 to 4, further comprising a displaying section which, also when the notification information is not output, is configured to display the identification information.

6. The monitoring apparatus according to claim 5, wherein
the identification information which is displayed on the displaying section identifies the own apparatus from the another monitoring apparatus, by means of at least one of a character, a symbol, and a color.

7. The monitoring apparatus according to any one of claims 1 to 6, further comprising an inputting section through which the identification information is input, wherein
the storing section updatably stores the identification information.
